# EUROPEAN PATENT APPLICATION

(11) **EP 0 224 283 A2**
(43) Date of publication of application: **03.06.1987**
(21) Application number: 86201713.4
(22) Date of filing: 12.05.1983
(51) Int. Cl.: C07D 213/80, C07D 211/90, C07D 401/12, C07D 501/36, A61K 47/00

(54) **Brain-specific drug delivery**

(30) Priority: 18.05.1982 US 379316; 27.01.1983 US 461543; 15.03.1983 US 475493
(62) Divisional of application: 83902034.4
(71) Applicant: UNIVERSITY OF FLORIDA, Gainesville, Florida 32611 (US)
(72) Inventor: Bodor, Nicholas S., Gainesville Florida 32608 (US)
(74) Representative: Pendlebury, Anthony

(57) **Abstract**

The invention provides compounds adapted for the site-specific/sustained delivery of a centrally acting drug species to the brain having the formula ID-DHC] and the non-toxic pharmaceutically acceptable salts thereof, wherein [D] is a centrally acting drug species containing at least one reactive -COOH, amide or imide functional group, and [DHC] is the reduced, biooxidizable, blood-brain barrier pentrating lipoidal form of a dihydropyridine 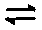 pyridinium salt redox carrier comprising a dihydropyridine ring system of the formula 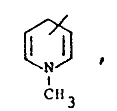
a ring carbon atom of the dihydropyridine ring system being connected via a bridging group to a reactive -COOH, amide or imide functional group in the centrally acting drug species. The corresponding quaternary derivatives are also described.

## Description

### Field of the Invention:

The present invention relates to a dihydropyridine/pyridinium salt type of redox system for the site-specific or sustained delivery (or both) of a wide variety of drug species to the brain. More especially, this invention relates to the discovery that a biologically active compound coupled to a lipoidal carrier moiety comprising a dihydropyridine nucleus readily and easily penetrates the blood-brain barrier ("BBB") and attains increased levels of concentration in the brain; oxidation of the dihydropyridine carrier moiety in vivo to the ionic pyridinium salts prevents its elimination from the brain, while elimination from the general circulation is accelerated, resulting in significant and pro- longedly sustained brain-specific drug activity, whether ascribable to the cleavage of the [D-QC]⁺ entity and sustained release of the drug in the brain and/or to (D-QC)⁺ itself.

### Background Art:

The delivery of drug species to the brain is ofttimes seriously limited by transport and metabolism factors and, more specifically, by the functional barrier of the endothelial brain capillary wall deemed the blood-brain barrier, BBB. Site-specific delivery and sustained delivery of drugs to the brain are even more difficult, and to date no useful simple or generic techniques to achieve such phenomena are known to the art.

Indeed, the barriers separating plasma from the brain and cerebrospinal fluid (CSF) are complex systems involving passive and active transport and subserve a number of important functions. The boundary between plasma and the central nervous system (CNS) is much less permeable than that between plasma and other tissue cells to a variety of water soluble substances, such as organic electrolytes, organic acids and bases, as well as to large molecules such as proteins. Such a barrier also provides a path for clearance from the brain of the breakdown products of cellular metabolism. The CNS and its fluids can be considered basically a three-compartment system: the blood or the plasma, CSF and brain tissue. There is a diffusion-controlled exchange between CSF and the extracellular fluid (CF) of the brain. It has also been suggested that the permeabilities of blood-CSF and blood-brain barriers are practically identical with respect to drugs and other foreign substances. Mayer et al, J. Pharmacol. and Ex^{p.} Therap., 125, 185 (1959).

The BBB is, moreover, basically the result of the fact that the endothelial cells in the brain capillaries are joined by continuous, tight intercellular junctions, such that material has to pass through the cells rather than between them in order to move from blood to brain. It is interesting that there are areas within the brain, such as the sub- fornical body and the postremia in which the capillary cells are not closely linked so that they lack the characteristics of the BBB. They provide the entry of small amounts of compounds which would not ordinarily enter the barriers. Hoffmann and Olszewzki, Neurology (Minneap.), 11, 1081 (1961).

Foreign compounds which enter organs other than the central nervous system with ease, may penetrate the CNS slowly or hardly at all. A number of theories concerning the nature of the barrier have been proposed. The widely accepted concept describes the boundary as a fat-like layer interspersed with small pores, although the BBB is not a simple, anatomically well-defined unitary physical entity. Shuttleworth, Prog. Exp. Tumor Res., 17, 279 (1972). Penetration of such a barrier may occur by several processes: lipid soluble substances may passively penetrate into the cells, while small molecules such as water and urea may pass through the pores. In addition to these simple physical processes, carrier- mediated and active transport processes govern the movement of many molecules through the BBB. Thus, it is generally accepted that lipid solubility, degree of ionic dissociation or protonation and the ability of temporary combination with membrane constituents affect delivery through the BBB. It has been shewn, for example, that in the class of barbiturates, a quantitative correlation could be established between their ease to pass into the brain (as reflected by the different times of onset of anesthetic action) and their lipid/water partition coefficient. Mark et al, J. Pharmacol. and Exp. Therap., 123, 79 (1957). The role of lipid solubility in drug penetration through the BBB is also exemplified by the better absorption of the sparingly water-soluble thiamine propyl disulfide (TPD) as compared to the water-soluble thiamine hydrochloride (THC1). Thomson et al, Ann. Int. Med., 74, 529 (1971). Some materials such as glucose and amino acids are transported by active mechanism, characterized by saturation, bidirectional molecular specificity, bidirectional competitive inhibition and bidirectional countertransport. Fishman, Am J. Physiol., 206, 836 (1964).

Changes in permeability of the BBB can be caused by several pathological and toxicological processes. Pardridge, Connor and Crawford, CRC Crit. Rev. Toxicol., 179 (1975). A general increase in the barrier permeability, such as a nonspecific breakdown of the barrier has, however, severe consequences, including cerebral edema.

It too is well documented that the BBB is relatively impermeable to the ionized forms of drugs and other molecules. Drugs which are weak organic electrolytes appear to pass from blood to CSF to reach a steady state ratio characteristic of each molecule according to its pK a and the existence of a normal pH gradient between blood and CSF. It is clear that it is the most difficult for quaternary pyridinium or ammonium salts to penetrate the BBB.

And removal of substances from the brain and CSF is obviously a significant factor in regulating drug concentrations in the CNS. There are several efflux processes: bulk flow via the arachnoid villi, diffusion of lipid soluble substances into brain and blood, active transport and metabolism by adjacent meninges. Once a drug or metabolite enters the CSF from blood or brain by simple diffusion, it may rapidly be removed, either by nonselective bulk flow or by active transport mechanism associated with the choroid plexus or other nondefined structures in the CSF compartment. It is generally accepted that highly lipid-soluble drugs leave the CSF more rapidly than poorly lipid-soluble ones, but the barrier to passage of compounds from CSF has only superficial similarity to the blood-CSF barrier.

Drug elimination processes from the brain are significantly directly related to drug accumulation in the brain. It is generally assumed that efflux in the opposite direction involves almost the same processes as for entry, except that the role of the bulk flow and the metabolic processes in the brain are not to be overlooked.

The two elimination processes studied in the earlier literature and which can be said to have a certain bearing on the present invention involve elimination from the brain of ionic species. Thus, it is found that non-metabolized ionic species, such as the acetate ion, have a three times slower elimination rate from the CSF than from the blood. Freundt, Arz., Forsch., 23, 949 (1973). An even more dramatic change in the elimination rate was found in the case of a quaternary piperidinium salt. The quaternary salt, formed in situ after delivery of a haloalkylamine, which undergoes cyclization to the quaternary salt, in the brain, as well, was found to have an at least ten times slower elimination rate from the brain than from the rest of the body. It was concluded by the authors (Ross and Froden, Eur. J. Pharmacol., 13, 46 [1970] that the outflow rate of the quaternary salt corresponded to the inflow rate. Similar results were obtained for the erythrocytes: the efflux of the quaternary salt was very slow. Ross, J. Pharm. Pharmacol., 27, 322 (1975).

And while it too has been suggested to deliver a drug species, specifically N-methylpyridinium-2-carbaldoxime chloride (2-PAM), into the brain, the active nucleus of which in and of itself constituting a quaternary pyridinium salt, by way of the dihydropyridine latentiated prodrug form thereof, such approach is conspicuously delimited to relatively small molecule quaternary pyridinium ring-containing drug species and does not provide the overall ideal result of brain-specific, sustained release of the desired drug, with concomitant rapid elimination from the general circulation, enhanced drug efficacy and decreased toxicity. Hence, no "trapping" in the brain of the 2-PAM formed in situ results, and obviously no brain-specific, sustained delivery occurs as any consequence thereof: the 2-PAM is eliminated as fast from the brain as it is from the general circulation and other organs. Compare U.S. Patents Nos. 3,929,813 and 3,962,447; Bodor et al, J. Pharm. Sci., 67, No. 5, 685 (1978). It has also been speculated to deliver, e.g., an antitumor agent into the brain by utilizing a dihydropyridine/pyridinium redox carrier moiety therefor, but this particular hypothesis necessarily entails derivatizing the dihydropyridine/pyridinium carrier with a substituent R₁ itself critically designed to control the release rate of the active drug species from the quaternary drivative thereof, as well as being critically functionally coordinated with the particular chemical and therapeutic activity/nature of the antitumor drug species itself; Bodor et al, J. Pharm. Sci., supra.

Accordingly, acutely serious need exists in this art for a truly effective generic but nonetheless flexible method for the site-specific, or sustained delivery, or both, of drug species to the brain, while at the same time avoiding the aforesaid noted and notable disadvantages and drawbacks associated with penetration of the blood-brain barrier, with dihydropyridine latentiated prodrug forms of drug species themselves comprising a pyridinium salt active nucleus, and with the necessity for introducing critically coordinated and designed, release rate-controlling substituents onto any particular drug carrier moiety.

Recently, Bodor et al, Science, Vol. 214, December 18, 1981, pp. 1370-1372, have reported on site-specific sustained release of drugs to the brain. The Science publication outlines a scheme for specific and sustained delivery of drug species to the brain, as depicted in the following Scheme 1:

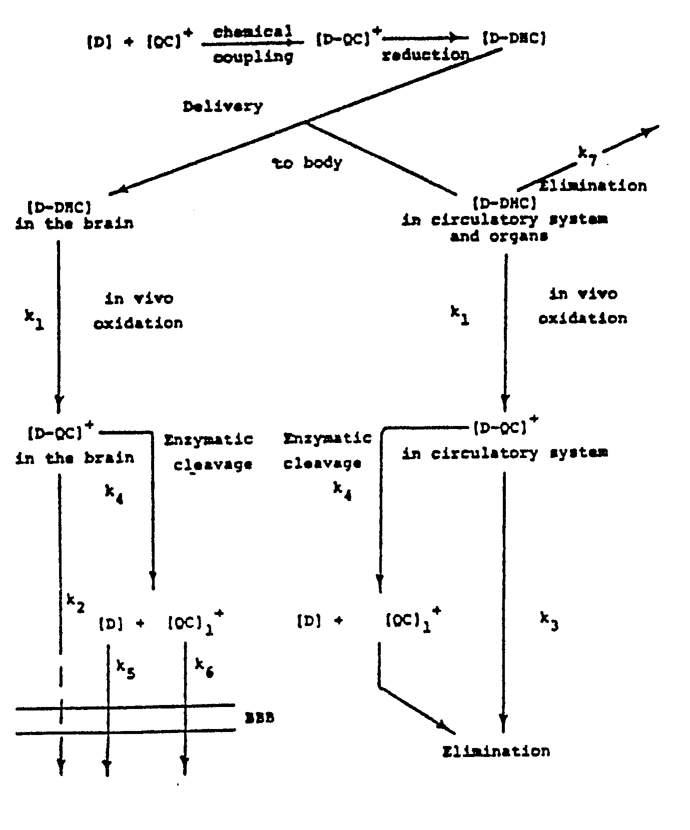

### scheme 1: BBB, blood-brain barrier.

According to the scheme in Science, a drug [D] is coupled to a quaternary carrier (QC]⁺ and the [D-QC]⁺ which results is then reduced chemically to the lipoidal dihydro form [D-DHC]. After administration of [D-DHC] in vivo, it is rapidly distributed throughout the body, including the brain. The dihydro form [D-DHC] is then in situ oxidized (rate constant, kₗ) (by the NAD 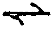 NADH system) to the ideally inactive original [D-QC]⁺ quaternary salt which, because of its ionic, hydrophilic character, should be rapidly eliminated from the general circulation of the body, while the blood-brain barrier should prevent its elimination from the brain (k ^{»} k₂; k₃ » ^{k}₇). Enzymatic cleavage of the [D-QCJ⁺ that is "locked" in the brain effects a sustained delivery of the drug species [D], followed by its normal elimination (k₅), metabolism. A properly selected carrier [QC]⁺ will also be rapidly eliminated from the brain (k₆ »k2). Because of the facile elimination of [D-QC]⁺ from the general circulation, only minor amounts of drug are released in the body (k₃ »k4); [D] will be released primarily in the brain (k₄ ^{>} k₂). The overall result ideally will be a brain-specific sustained release of the target drug species.

Bodor et al have reported,in Science, their work with phenylethylamine as the drug model, which was coupled to nicotinic acid, then quaternized to give compounds of the formula 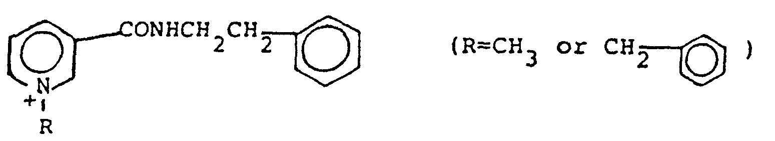 which were subsequently reduced by sodium dithionite to the corresponding compounds of the formula 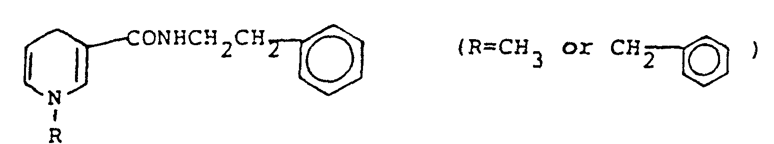 Testing of the N-methyl derivative in vivo supported the criteria set forth in Scheme 1. Bodor et al speculated that various types of drugs might possibly be delivered using the depicted or analogous carrier systems and indicated that use of N-methylnicotinic acid esters and amides and their pyridine ring- substituted derivatives was being studied for delivery of amino- cr hydroxyl-containing drugs, including small peptides, to the brain. No other possible specific carriers were disclosed.

Other reports of Bodor et al's work have appeared in The Friday Evening Post, August 14, 1981, Health Center Communications, University of Florida, Gainesville, Florida; Chemical & Engineering News, December 21, 1981, pp. 24-25; and Science News, January 2, 1982, Vol. 121, No. 1, page 7. These publications do not suggest any carrier systems other than the specific N-methyl and N-benzyl nicotinic acid-type carriers disclosed in the Science publication. Other classes of drugs as well as a few specific drugs are mentioned as possible candidates for derivativization; for example, steroid hormones, cancer drugs and memory enhancers are indicated as targets for possible future work, as are enkephalins, and specifically, dopamine and testosterone. The publications do not suggest how to link such drugs to the carrier, except possibly when the drugs are simple structures containing a single NH₂ or, perhaps, simple structures containing a single OH, of the primary or secondary type, as is the case with phenylethylamine or testosterone. There is, for example, no suggestion of how one of ordinary skill in the art would form a drug-carrier combination when the drug has a more complicated chemical structure than phenylethylamine, e.g., dopamine or an enkephalin. Thus, except in a very limited area, where only a combination of certain types of drug structures with certain utility classes and certain carrier structures is taught or suggested, the publications simply do not make the broad concept of the invention as represented by Scheme 1 available to the public. Likewise, the many embodiments of the invention as described hereinafter are unsuggested by the art.

It is also known to this art that Parkinsonism, a striatal dopamine deficiency syndrome [H. Ehringer and O. Hornykiewicz, Klin. Wsch., 38, 1236 (1960)], cannot be treated directly with dopamine, for dopamine and related catecholamines also do not cross the blood-brain barrier [B. E. Roos and G. Steg, Life Sci., 3, 351 (1964)]. L-Dopa, considered as a prodrug for dopamine, was first discovered to be useful in the treatment of Parkinsonism more than twenty years ago [A. Barbeau, Exce^{p}ta Medica, Int. Congr. Ser., 38, 152 (1961); W. Birkmayer and O. Hornykiewicz, Wien. Klin. Wochnenschr., 73, 787 (1961)). Indeed, L-Dopa is considered to be the best available treatment for Parkinsonism, but, unfortunately, at the expense of a wide variety of undesirable side effects [A.Barbeau, TIPS, 2, (11), 297 (1981)]. The peripheral side effects of L-Dopa, which range from nausea and vomiting to cardiac arrythmias and hypotension, appear to be due to one or more of the metabolic products thereof, rather than L-Dopa per se. L-Aromatic amino acid decarboxylase enzyme is responsible for the major metabolism of L-Dopa, whether prior, during or after absorption. Concurrent administration of L-Dopa with an inhibitor of aromatic amino acid decarboxylase, which should not be able to penetrate the BBB, reduces the decarboxylation of L-Dopa in peripheral tissues. Such reduction allows higher proportions of L-Dopa to reach the CNS and at the same time diminishes the peripheral side effects considerably, particularly vomiting and cardiac arrythmias, but a number of serious side effects still persist [A.Barbeau, TIPS supra; A.Barbeau and M.Roy, Neurology, 26, 399 [(1976)]. Attempts have also been made to alleviate the well-known dissolution, absorption and metabolism problems of L-Dopa [H. Ninterberger, Biochem. Med., 5, 412 (1971); H. Shindo, T. Komai, K. Tanaka, E. _{N}akajima and N. Miyakoshi, Chem. Pharm. Bull., 21, 826 (1973); C.O. Rutledge and M.M. Hoehn, Nature (London), 244, 447 (1973); R._{L}. Bronaugh, R.J. McMurty, M.M. Hoehn and C.O. Rutledge, Biochem. Pharmacol., 24, 1317 (1975)], employing prodrug approaches [N. Bodor, _{K}.B. Sloan, T. Higuchi and K. Sasahara, J. Med. Chem., 20, 1435 (1977); A.M. Felix, D.P. Winter, S.S. Wing, I.D. Kulesha, W.R. Pool, D.L. Hane and H. Sheppard, J. Med. Chem., 17, 422 (1974)].

Additionally, dopamine agonists, which are used in the treatment of hyperprolactinemia associated with pituitary adenomas or amenorrhea [R.F. Spark and G. Dickenstein, Ann. Int. Med., 90, 949 (1979)], also induce unwanted side effects.

Thus, especially acutely serious need exists in this art to deliver a dopaminergic agent directly and specifically to the brain, in a sustained manner, and there elicit the desired dopaminergic response, e.g., for the treatment of Parkinsonism or hyperprolactinemia.

### Summary and Objects of the Invention:

Accordingly, a major object of the present invention is the provision of a generic method for the specific and/or target enhanced delivery to the brain of a wide variety of drug species and to achieve brain-specific drug delivery by effecting the bidirectional transport of the drug species into and out of the brain employing dihydropyridine pyridinium salt carrier type redox systems.

Another object of the invention is to provide for brain specific drug delivery utilizing a dihydropyridine 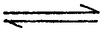 pyridinium salt carrier type redox system, which drug/carrier system is characterized by enhanced drug efficacy and decreased toxicity. Indeed, consistent herewith systemic toxicity is significantly reduced by accelerating the elimination of the drug/quaternary carrier system, and even central toxicity is reduced by providing a low level, sustained release of the active drug species in the brain.

Yet another object of this invention is the provision of a chemical delivery system for the site-specific and sustained release of drug species to the brain, and one in which a special pro-prodrug reduced form of an active drug species is actually delivered to the body of a patient, not a prodrug as such and not a drug/carrier entity necessarily comprised of critically tailored release rate-controlling substituent(s).

Yet another object of this invention is to provide enhanced delivery to the brain of a wide variety of centrally acting agents which are not themselves able to penetrate the blood-brain barrier to any considerable extent.

In accord with the foregoing, the present invention provides compounds adapted for the site-specific/sustained delivery of a centrally acting drug species to the brain, said compounds being:
compounds of the formula 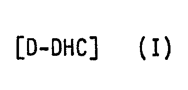
and the non-toxic pharmaceutically acceptable salts thereof, wherein [D] is a centrally acting drug species containing at least one reactive -COOH, amide or imide functional groups, and [DHC] is the reduced, biooxidizable, blood-brain barrier penetrating lipoidal form of a dihydropyridine 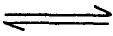 pyridinium salt redox carrier comprising a dihydropyridine ring system of the formula 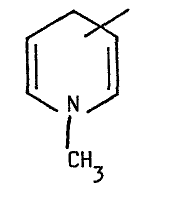 a ring carbon atom of the dihydropyridine ring system being connected via a bridging group to a reactive -COOH, amide or imide functional group in the centrally acting drug species.

In another aspect, the present invention provides compounds having the formula 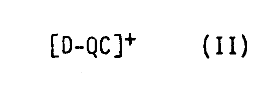 wherein [D] is a centrally acting drug species containing at least one reactive -COOH, amide or imide functional group, and [QC]⁺ is the hydrophilic, ionic pyridinium salt form of a dihydropyridine 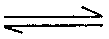 pyridinium salt redox carrier comprising a pyridinium ring system of the formula 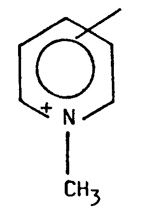 a ring carbon atom of the pyridinium ring system being connected via a bridging group to a reactive -COOH, amide or imide functional group in the centrally acting drug species.

### Detailed Description of the Invention:

More particularly in accord with the present invention, the following definitions are applicable:
The term "lipoidal" as used herein is intended to designate a carrier moiety which is lipid-soluble or lipophilic.

The expression "hydroxyl protective group" is intended to designate a group which prevents premature metabolism of an OH group or groups prior to the compound's reaching the desired site in the body. Typical hydroxyl protective groups contemplated by the present invention are acyl groups and carbonates.

khen the hydroxyl protective group is acyl (i.e., when it is an organic radical derived from a carboxylic acid by removal of the hydroxyl group), it preferably represents an acyl radical selected from the group consisting of alkanoyl having 2 to 8 carbon atoms; alkenoyl having one or two double bonds and 3 to 8 carbon atoms; 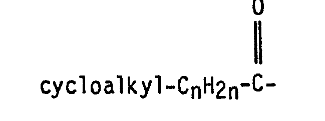 wherein the cycloalkyl portion contains 3 to 7 ring atoms and n is zero, one, two or three; phenoxyacetyl; pyridinecarbonyl; and 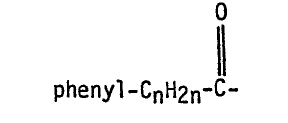 wherein n is zero, one, two or three and phenyl is unsubstituted or is substituted by 1 to 3 alkyl each having 1 to 4 carbon atoms, alkoxy having 1 to 4 carbon atoms, halo, trifluoromethyl, dialkylamino having 2 to 8 carbon atoms or alkanoylamino having 2 to 6 carbon atoms.

when the acyl group is alkanoyl, there are included both unbranched and branched alkanoyl, for example, acetyl, propionyl, butyryl, isobutyryl, valeryl, isovaleryl, 2-methylbutanoyl, pivalyl (pivaloyl), 3-methylpentanoyl, 3,3-dimethylbutanoyl, 2,2-dimethylpentanoyl and the like. Pivalyl and isobutyryl are especially preferred.

When the acyl group is alkenoyl, there are included, for example, crotonyl, 2,5-hexadienoyl and 3,6-octadienoyl. 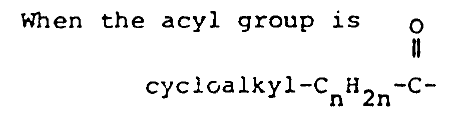 there are included cycloalkanecarbonyl and cyclo- alkanealkanoyl groups wherein the cycloalkane portion can optionally bear 1 cr 2 alkyl groups as substituents, e.g. cyclopropanecarbonyl, 1-methylcyclcpropanecarbonyl, cyclopropaneacet^{y}l, a-methylcyclopropaneacetyl, 1-methylcyclopropaneacetyl, cyclopropanepropionyl, a-methylcyclopropanepropionyl, 2-iscbutylcyclopro- panepropionyl, cyclobutanecarbonyl, 3,3-dimethylcyclo- butanecarbonyl, cyclobutaneacetyl, 2,2-dimethyl-3-ethylcyclobutaneacetyl, cyclopentanecarbonyl, cyclohexaneacetyl, cyclcheptanecarbonyl and cyclo- heptanepropionyl.

When the acyl group is pyridinecarbonyl, there are included picolinoyl (2-pyridinecarbonyl), nicotinoyl (3-pyridinecarbonyl) and isonicotinoyl (4-pyridinecarbonyl).

When the acyl group is 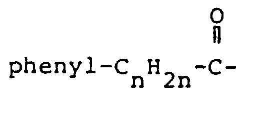 there are included, for example, benzoyl, phenylacetyl, a-phenylpropionyl, B-phenylpropionyl, p-toluyl, m-toluyl, o-toluyl, o-ethylbenzoyl, p-tert-butylbenzoyl, 3,4-dimethylbenzoyl, 2-methyl-4-ethylbenzoyl, 2,4,6-trimethylbenzoyl, m-methylphenylacetyl, p-isobutyl- phenylacetyl, S-(p-ethylphenyl)-propionyl, p-anisoyl, m-anisoyl, o-anisoyl, m-isopropoxybenzoyl, p-methoxyphenylacetyl, m-isobutoxyphenylacetyl, m-diethyl- aminobenzoyl, 3-methoxy-4-ethoxybenzoyl, 3,4,5-trimethoxybenzoyl, p-dibutylaminobenzoyl, p-n-butoxy- benzoyl, 2,4,6-triethoxybenzoyl, 3,4-diethoxyphenyl- acetyl, S-(3.4,5-trimethoxyphenyl)propionyl, o-iodobenzoyl, m-bromobenzoyl, p-chlorobenzoyl, p-fluorobenzoyl, 2-bromo-4-chlorobenzoyl, 2,4,6-trichlorobenzoyl, p-chlorophenylacetyl, α-(m-bromophenyl)propionyl, p-trifluoromethylbenzoyl, 2,4-di(trifluoromethyl)benzoyl, m-trifluoromethyl- phenylacetyl, S-(p-trifluoromethylphenyl)propionyl, 2-methyl-4-methoxybenzoyl, 3-chloro-4-ethoxybenzoyl, β-(3-methyl-4-chloroph,enyl)propionyl, p-dimethylaminobenzoyl, p-(N-methyl-N-ethylamino)benzoyl, o-acetamidobenzoyl, m-propionamidobenzoyl, 3-chloro-4-acetamidophenylacetyl and p-acetamidophenylpropionyl.

When the hydroxyl protective group is a carbonate grouping, it has the structural formula 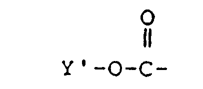 i.e., it is an organic radical which can be considered to be derived from a carbonic acid by removal of the hydroxyl group from the COOH portion. Y' preferably represents alkyl having 1 to 7 carbon atoms; alkenyl having one or two double bonds and 2 to 7 carbon atoms; 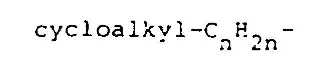 wherein the cycloalkyl portion contains 3 to 7 ring atoms and n is zero, one, two or three; phenoxy; 2-, 3- or 4-pyridyl; or 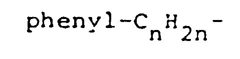 wherein n is zero, one, two or three and phenyl is unsubstituted or is substituted by 1 to 3 alkyl each having 1 to 4 carbon atoms, alkoxy having 1 to 4 carbon atoms, halo, trifluoromethyl, dialkylamino having 2 to 8 carbon atoms or alkanoylamino having 2 to 6 carbon atoms. Most preferably, Y' is Cₗ-C₇ alkyl, particularly ethyl or isopropyl.

Similarly, the expression "carboxyl protective group" is intended to designate a group which prevents premature metabolism of a COO_{H} group or groups prior to the compound's reaching the desired site in the body. Typical carboxyl protecting groups are the groups encompassed by Y' above, especially Cₗ-C₇ alkyl, particularly ethyl or isopropyl.

By "Centrally acting" drug species, active agent or compound as utilized herein, there is of course intended any drug species or the like, the principal pharmacological activity of which is CNS and a result of direct action in the brain.

Exemplary such centrally acting drug species are the CNS-amines and other nervous system agents, whether sympathetic or parasympathetic, e.g., L-DOPA, muscle relaxants, tranquilizers and antidepressants, e.g., benzodiazepine tranquilizers such as oxazepam, phenothiazine tranquilizers, mild and strong analgesics and narcotics, sedatives and hypnotics, narcotic antagonists, vascular agents, stimulants, anesthetics, small peptides, such as the di-, tri, tetra-and pentapeptides, and other small 6-20 aminoacid unit containing peptides, e.g., the enkephalins (for example, Tyr-Gly-Gly-Phe-Leu), which, besides being analgesics, initiate epileptic activity in the brain at doses that are about tenfold lower than for effecting analgesic activity, larger peptides, such as pituitary hormones and related agents, anti-epileptic and anticonvulsant drugs generally, including hydantoins such as phenytoin and ethotoin, barbiturates such as phenobarbital, hormones, amphetamine-like drugs, anticancer and anti-Parkinsonism agents, antihypertensives, agents to enhance learning capacity and the memory processes, including treatment of dementias, such as Alzheimer's disease, antibacterials, centrally active hypotensive agents, diagnostic agents, such as radio-pharmaceuticals, monoamine oxidase (MAO) inhibitor drugs, CNS or brain important/essential amino acids, such as tryptophan, and any like centrally acting compounds.

Other illustrative ultimate species of centrally active drug entities are: methyldopa, which is a sympatholytic agent used, e.g., in hypertension; clorazepate and nitrazepam, which are benzodiazepine-type tranquilizers; ethotoin, phenobarbital and aminoglutethimide, anticonvulsants; bemegride, a barbiturate antagonist; iodopyracet, iodouppurate, iodamide and iopanoic acid, which are radiodiagnostics; amoxicillin, oxacillin, carbenicillin and ampicillin, pencillin-type antibiotics; amobarbital, a sedative; chlortetracycline, tetracycline and methacycline, which are tetracycline-type antibiotics; clindamycin, lincomycin, nalidixic acid and oxolinic acid, antibacterials/antibiotics; bromazepam and lorazepam, tranquilizers; phenytoin, an anticonvulsant; glutethimide, a mild hypnotic/sedative; methyprylon; dicloxacillin, an antibacterial; butalbital, a barbiturate sedative; GABA, _{Y}-vinyl GABA, _{f}-acetylenic GABA, neurotransmitters for possible use in epilepsy; valproic acid and its metabolites such as 5-hydroxy-2-n-propyl- pentanoic acid, 4-hydroxy-2-n-propylpentanoic acid, 3-hydroxy-2-n-propylpentanoic acid, for use as anticonvulsants; methotrexate, podophyllotoxin derivatives and cyclophosphamide, anticancer/antitumor agents; cephalothin, cephalexin and cefoxitin, cephalosporin antibiotics; atenolol a B-blocker/hypotensive; LH-RH, a neurotransmitter; ribavirin and acyclovir, antiviral agents; chlorambucil and melphalan, nitrogen mustard-type anticancer/antitumor agents; methotrexate and aminopterin, which are folic acid antagonist-type anticancer/antitumor agents; cisplatin-analogue type anticancer/antitumor agents; mitomycin C, which is used in cancer chemotherapy; hydroxyurea and DON, anticancer urea derivatives; and N,N'-bis-(dichloracetyl)-1,8-octamethylene diamine, an agent for male fertility inhibition.

Preferred classes of centrally acting drugs for use herein are the central neurotransmitters, anticancer and antitumor agents, antiviral agents, tranquilizers, memory enhancers and hypotensives. Among the neurotransmitters, there can be mentioned amino acids, such as GABA, glycine, glutamic acid and aspartic acid; and peptides such as neurotensin, luteinizing hormone-releasing hormone (LHRH), enkephalins such as met⁵⁻enkephalin and leu⁵⁻enkephalin, endorphins such as _{Y}-, a- and 6-endorphins, oxytocin M and vasopressin. Among the anticancer and antitumor agents, there can be mentioned 3-deazaguanine, tiazofurin, sangivamycin, PCNU, spiromustine, L-alanosine, DON, L-ICRF, 5-methyl tetrahydrohomofolic acid, glyoxylic acid sulfonylhydrazone, DACH, SR-2555, SR-2508, bactobolin, acivicin, hydroxyurea, chlorambucil, uracil mustard, melphalan, 5-FUDR, methotrexate, aminopterin, mitomycin C, and podopnyllotoxin derivatives. Among the antiviral agents, there can be mentioned ribavirin; acyclovir; diarylamidines; 2-aminoxazoles; benzimidazole analogues; bridgehead C-nucleosides; phenyl glucoside derivatives; and 6-azauridine. Among the tranquilizers, there can be mentioned benzodiazepine tranquilizers such as oxazepam, lorazepam, chlordiazepoxide, bromazepam, clorazepate and nitrazepam; hydantoin-type tranquilizers such as phenytoin, ethotoin, mephenytoin; phenothiazine-type tranquilizers; and others. Among the hypotensives, there can be mentioned methyldopa.

It too will be appreciated that by "dihydropyridine carrier" or "[DHC]", there is intended any nontoxic carrier moiety as hereinbefore defined, comprising, containing or including the dihydropyridine nucleus, the only criterion therfor being capacity for BBB penetration and in vivo oxidation tnereof to the corresponding quaternary pyridinium salt carrier [QC]⁺. As aforesaid, the ionic pyridinium salt drug/carrier prodrug entity [D-QC]⁺ which results from such in vivo oxidation is prevented from efflux from the brain, while elimination from the general circulation is accelerated. Subsequently, the covalent or equivalent bond coupling the drug species [D] to the quaternary carrier [QC]⁺ is metabolically cleaved which results in sustained delivery of the drug [D] in the brain and facile elimination of the carrier moiety [QC]^{+.} Such "covalent or equivalent bond" between the drug and the quaternary carrier is comprised of a linking group or function, necessitated because the drug species is not susceptible to direct chemical coupling to either the dihydropyridine carrier or the quaternary carrier. The cleavage of the [D-QC]⁺ prodrug to sustaineedly deliver the drug species [D] in the brain with concomitant facile elimination of the carrier moiety [Q_{C]}⁺ is characteristically enzymatic cleavage, e.g., by esterase, amidase, cholinesterase, hydrolytic enzyme, or peptidase, albeit any type of in brain cleavage which might result, whether enzymatic, metabolic or otherwise, of course remains within the ambit of this invention. Thus, the drug release rate controlling parameter of the subject pro-prodrugs is imparted simply via the cleavable bonding between drug and carrier, and not by any release rate controlling substituent(s).

The expression "non-toxic pharmaceutically acceptable salts" as used herein generally includes the nontoxic salts of compounds of formula (I), wherein [D] is a centrally acting drug species and [DHC] is the reduced, biooxidizable, blood-brain barrier penetrating form of a dihydropyridine - pyridinium salt redox carrier as hereinbefore defined formed with nontoxic, pharmaceutically acteptable inorganic or organic acids HX. For example, the salts include those derived from inorganic acids such as hydrochloric, hydrobromic, sulfuric, sulfamic, phospnoric, nitric and the like; and the salts prepared from organic acids such as acetic, propionic, succinic, glycollic, stearic, lactic, malic, tartaric, citric, ascorbic, pamoic, maleic, hydroxymaleic, phenylacetic, glutamic, benzoic, salicylic, sulfanilic, fumaric, methanesulfonic, toluene-sulfonic and the like.

The present invention provides a flexible arsenal of dihydropyridine pyridinium salt redox carriers for the site-specific/sustained delivery of virtually any centrally acting drug species as defined herein to the brain. Moreover, any dihydropyridine/pyridinium salt redox carrier entity is contemplated and intended hereby generically, and any such carrier moiety need not be, and is not derivatized with a drug release rate controlling substituent critically tailored to meet, or be coordinated with, the chemical nature and delivery requirements of the particular drug species sought to be preferentially administered to the brain. As utilized herein, the term "carrier" is to be understood as connoting just such a non-derivatized, non-drug/carrier coordinated entity, for consistency herewith it is the "carrier" entity itself and not the nature of any activity or release rate controlling/modifying substituent which is responsbible for providing the desired brain-specific or testicular-specific result.

Examples of such redox carriers include the quaternary pyridinium alcohols (1), and multi-charged delivery forms, exemplified by structure 2 (D represents drug, Z a covalent link) and obviously the corresponding dihydro forms.

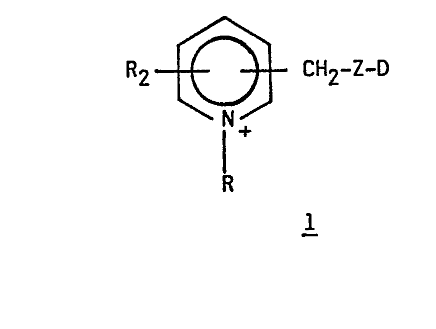

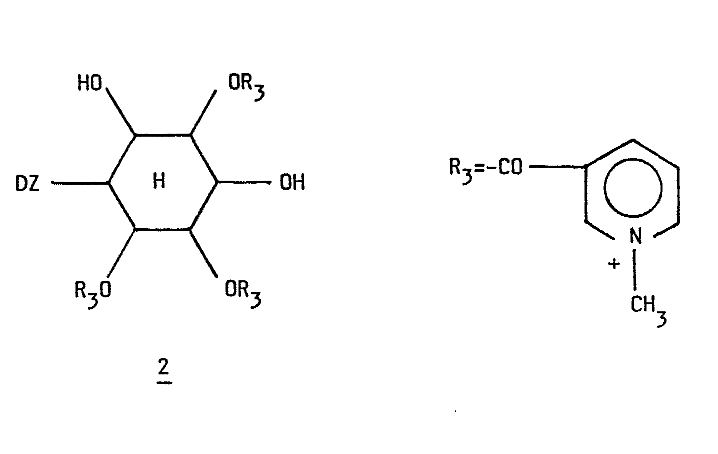

Naturally, selection of the particular dihydropyridine 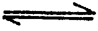 pyridinium salt redox carrier to be used will depend on the chemical structure of the specific drug involved. And not only should the nature of the functional group which is to be linked to the carrier system be considered in selecting the carrier, but the manner in which the ultimate compound is prepared should be tailored to the presence of any other reactive groups in the molecule. The following examples of specific drug/carrier combinations and their manner of synthesis are set forth for the purpose of illustration only and are not to be considered limitative in any way whatsoever.

One specific illustration utilizes chlorambucil as the target drug, in which case the desired nicotinic acid carrier system is linked to the drug via a bridging group. Thus, nicotinic acid can be reacted witn an appropriate di- or polyhydroxy compound such as ethylene glycol, propylene glycol or inositol and the resultant intermediate is linked via its free hydroxy group(s) to the carboxylic acid function of chlorambucil. That intermediate is then quaternized and the quaternary salt is reduced to afford the chlorambucil-CDS. In the case of nicotinic acid and ethylene glycol starting materials, the chlorambucil-CDS has the formula

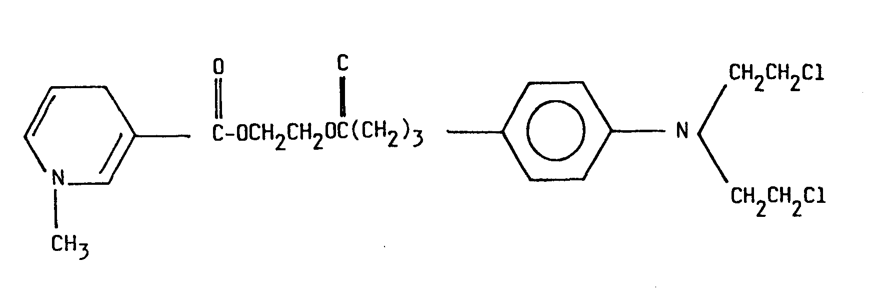

On the other hand, when a polyhydroxy compound is reacted with nicotinic acid in the first step, a variety of products are possible. Thus, for example, when inositol is used, the final product may contain anywhere from 1 carrier/5 drug residues to 5 carrier/l drug residue. In the case of the inositol trinicotinate intermediate 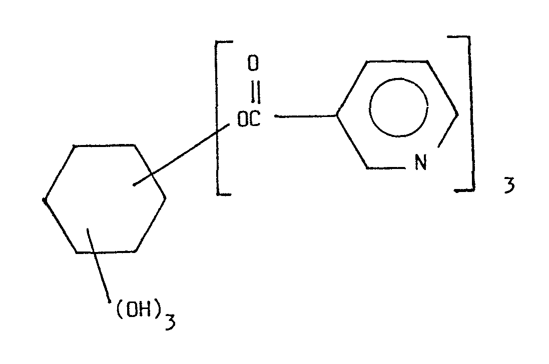 conditions for reacting same with chlorambucil can be selected so that one, two or three of the hydroxy functions react with the acid. When all three hydroxys react, the ultimate chlorambucil-CDS has the formula 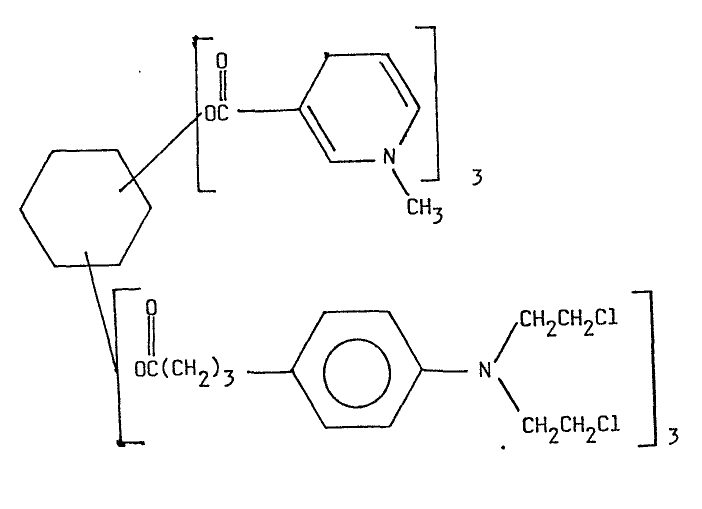 and contains 3 drug residues and 3 carrier groupings.

As another example, methotrexate, which has the structural formula 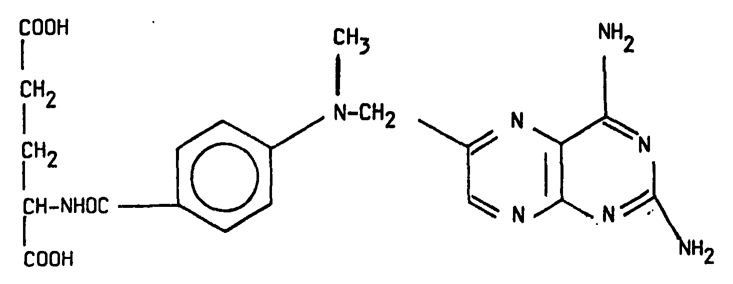 can be derivatized similarly to chlorambucil via its carboxy function(s), e.g., utilizing the inositol trigonellinates or a glucosamine analogue.

Known cisplatin analogues, in which typically the amino groups have been replaced with organic radicals, can be similarly derivatized according to the invention, the method of choice depending on the nature of the functional groups in the organic radicals.

Similarly, syntheses and like determinations as regards the redox carrier-linked enkephalins can be carried out, e.g. the leucinol trigonelline ester XV and its dihydro derivative XVI.

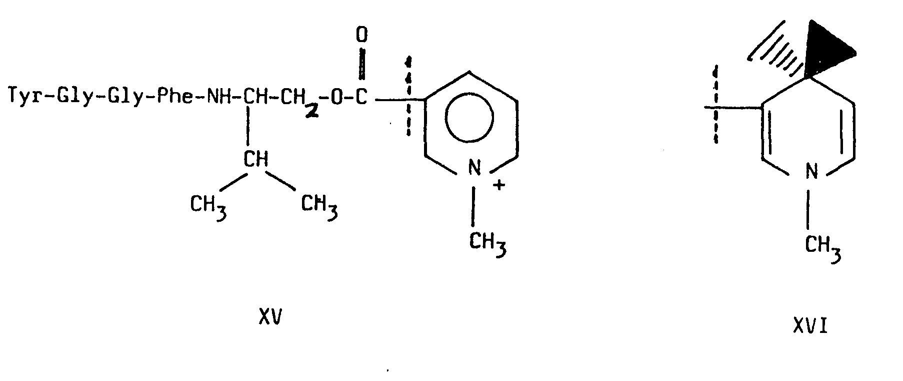

Thus, the site-specific brain delivery of the enkephalins for the treatment of epilepsy is established consistent with the Scheme 1, as is their analgesic activity.

And in another preferred embodiment of the invention, there is provided the effective, selective and nontoxic treatment of epilepsy, based upon the mechanism illustrated in Scheme 1. Indeed, commencing from the "GABA-hypothesis" of epilepsy, the brain-specific, enhanced and sustained release of GABA (y-aminobutyric acid) itself, and various other compounds either directly or indirectly affecting the concentrations of GABA in the brain, is circumscribed consistent herewith. Model compounds include carboxylic acids, most specifically valproic acid, as well as some of the GABA analogs which inhibit irreversibly the GABA-T, such as y-vinyl and/or y-acetylenic GABA. Using the aforesaid trigonelline (N-methylnicotinic acid) dihydrotrigonelline system, for example, the selected compounds can be effectively delivered per Scheme 1.

In the case of valproic acid, some alternatives are: 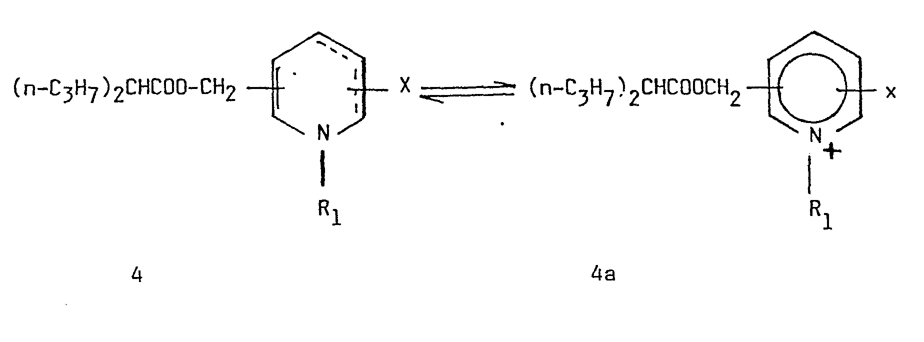 X = H, CONH₂, CHNOR₂, etc. Rₗ = CH₃, C₃H₇ or CH₂C₆H₅

In another embodiment of like delivery system, applicable for both the GABA and related compounds and for the carboxylic acids, or for any other drug species to be linked to such a carrier, either directly or indirectly, i.e., mediated by a carboxylic acid, e.g., succinic acid, or other linkage, provided is a mono- or poly-substituted nontoxic polyol (such as inositol or sugars) having the trigonelline 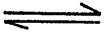 dihydrotrigonel line system and the compounds to be delivered linked to the same molecule as exemplified by the GABA case (5 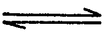 5a) and valproic acid (6 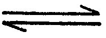 6a): 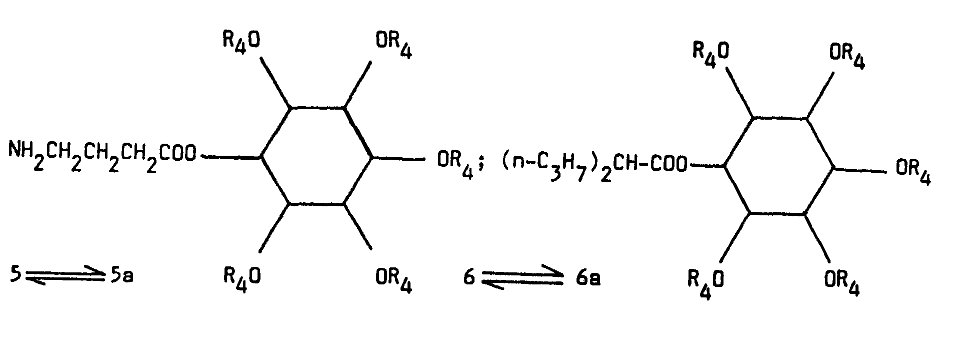 R₄ = H, GABA or valproic acid, but at least one of R₄ is: 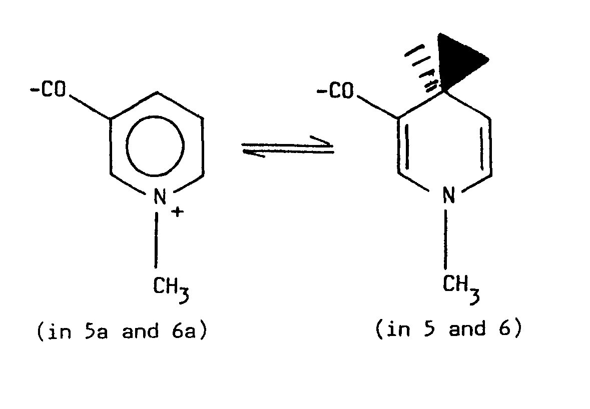 R₄ can be partially replaced by additional GABA or valproic acid, changing the carrier/drug ratio as necessary.

Based upon the observation that NADH content is significantly reduced in epileptic and like seizures, the use of the subject redox system (in reduced form) will bias the NAD 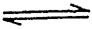 NADH balance towards NADH during the dihydro carrier > quaternary transformation. Also, the brain-specific delivery of small peptides consistent herewith, e.g., the enkephalins which have been found to initiate epileptic seizures, has led to the design of a variety of long lasting potent antagonists.

And the subject chemical delivery system is also useful for the delivery of other anticonvulsants in a sustained, brain-specific fashion, e.g., the benzodiazepines and hydantoins.

It will of course be appreciated in the immediately above regard that the drug treatment of epilepsy has always posed formidable problems. There are many different anticonvulsants available, some more specific for different types of seizures. Indeed, there exist a wide variety of opinions as to which is the most suitable drug for any particular type of seizure, and drug mixtures are typically employed. An inevitable result of the traditional,therapy is the development of chronic toxicity, but such result is conspicuously avoided according to the present invention.

It will be appreciated that the desired therapeutic effects of all antiepileptic agents investigated, as well as their undesired toxic effects, reflect a statistically significant correlation with the drug levels in plasma. This correlation is based upon a close relationship between the drug concentrations in plasma and brain tissue. In contrast, a primary attribute of this invention is to enable attainment of high and sustained brain levels of the selected active agents, essentially against the plasma-brain concentration gradient and independent of the drug concentration in the blood.

GABA and related compounds are logical candidates. It has been shown that GABA neuron function is impaired in at least certain types of human epilepsy. Animal studies also showed that seizures are induced by reduction of GABA neuron function to a critical degree by (1) inhibition of GABA synthesis, (2) blockade of GABA receptors or (3) inhibition of GABA-receptor mediated ionic events. In addition, enhancement of GABA synaptic activity (by direct receptor stimulation or by increasing GABA levels in the synapse) has a potent and wide spectrum anticonvulsant effect. These findings foreshadowed that an enhanced and sustained GABA brain delivery or a brain-specific delivery in a sustained manner of a good GABA-agonist would be efficacious in different forms of epilepsy. It is well known that GABA itself, when administered systematically, does not penetrate the normal blood-brain barrier to any significant extent. Among the potential sites at which drugs may act to influence GABA-mediated synaptic function, the first target is to effect the BBB transfer of GABA via a redox delivery system. The second main target is to effect the catabolizm of GABA. This invention, accordingly, specifically provides for the efficacious delivery of the GABA-T inhibitors, y-vinyl and y-acetylene-GABA, but the delivery of valproic acid, specifically to the brain and without requiring high circulating blood levels, is also envisaged. In order to achieve the required activity, sodium valproate must have a relatively high, 50-100 ug/ml, level in the blood. The value of valproic acid is well established in most types of epilepsy. It is evident that valproic acid produces significant increases in both brain and synaptosomal GABA concentrations. Valproic acid itself undergoes extensive metabolism.

In capsule summary, the present invention provides for the significantly improved treatment of epilepsy, and concomitant reduction in toxicity of a number of antiepileptic drug species currently in use. And made available to the brain is a variety of important compounds, such as GABA and a wealth of GABA-ergic agents.

It will be apparent from the foregoing discussion of specific drug-carrier combinations that a wide variety of synthetic approaches can be utilized, depending on the chemical nature of the selected drug. Various illustrative synthetic schemes as applied to specific centrally acting drugs in accord with this invention are set forth below in the section entitled "Illustrative Synthetic Methods". While the sequence of reaction steps can be varied in many cases, in general, the final step (except in the case of optional salt formation) will be reduction of a pyridinium compound of formula (II) to the corresponding dihydropyridine compound of formula (I). The reduction is usually conducted at a temperature from about -10°C to room temperture, for a period of time from about 10 mins to 2 hrs. conveniently at atmospheric pressure. Typically, a large excess of reducing agent is employed, e.g., a 1:5 molar ratio of reducing agent to starting [D-QC]⁺ compound. The process is conducted in the presence of a suitable reducing agent, preferably an alkali metal dithionite such as sodium dithionite or an alkali metal borohydride such as sodium borohydride or lithium aluminum borohydride, in a suitable solvent. Sodium dithionite reduction is conveniently carried out in an aqueous solution; the dihydro product [D-DHC] is usually insoluble in water and thus can be readily separated from the reaction medium. In the case of sodium borohydride reduction, an organic reaction medium is employed, e.g., a lower alkanol such as methanol, an aqueous alkanol or other protic solvent.

Suitable nontoxic pharmaceutically acceptable carriers (i.e. diluentes or vehicles) for use with the topic compounds [D-DHC], e.g., those less toxic than the target drug species themselves, will be apparent to those skilled in this art. Compare, for example, Remington's Pharmaceutical Sciences, 4th Edition (1970). Obviously, the choice of suitable carriers (i.e. diluentes or vehicles) will depend upon the exact nature of the particular dosage form selected, as well as upon the identity of the active drug species [D]. The therapeutic dosage ranges for administration of the compounds according to this invention will generally be the same as, or less than, those characteristically used in this art for administration of the known drug species [D], per se. Naturally, such therapeutic dosage ranges will vary with the size of the patient, the condition for which the [D-DHC] compound is administered, the particular dosage form employed, and the like. The quantity of given dosage form needed to deliver the desired dose of [D] will of course depend upon the concentration of [D-DHC] in any given pharmaceutical composition/dosage form thereof.

The ability of the topic compounds to cross the BBB and to be "locked into" the brain allows administration of the drug in a site-specific manner. A combination of the present dihydropyridine 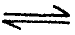 pyridinium salt redox system with a sustained release system will further enhance this site-specificity. Thus, a preferred embodiment of the invention comprises formulating the [D-DHC] compound or the salt of a [D-DHC] compound utilizing a sustained release carrier (i.e. diluente or vehicle) system and/or route of administration capable of slowly releasing the chemical, e.g., sustained release tablets and capsules for oral administration; subcutaneous injection, or implantation of drugs in solid pellet form (for example, distributed in a biodegradable polymer); intramuscular injection of the compound in solution in oil or suspended in a repository vehicle; a transdermal delivery device or form such as an ointment to be applied locally to the desired site (when the drug is susceptible of delivery through the skin) and the like. The rate of release of compound from the sustained release system should be comparable to the rate of in vivo oxidation of the dihydro form of the redox system in order to achieve the greatest degree of enhancement of specificity.

### 1. Methods for Derivatizing -NH₂ or -NH- Groups Which Are Part of Amide or Imide Functions in Drugs

### Method A

The drug is first reacted with an aldehyde (e.g. formaldehyde, benzaldehyde, acetaldehyde or chloral (Cl₃CCHO)); for Example, in the case of chloral, one converts the -NH- function to a 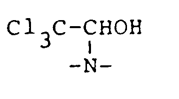 function and thus forms a suitable bridging group. The resultant compound is then reacted with nicotinic acid in the presence of a suitable dehydrating agent, or with nicotinoyl chloride or nicotinic anhydride, to form the corresponding nicotinic acid ester of the partial formula 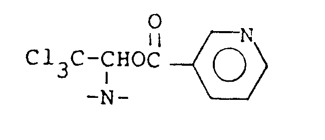 The resultant intermediate is then quaternized, typically by treatment with methyl iodide in a suitable organic solvent, to afford the quaternary derivative [D-QC]⁺ which is then reduced by treatment with sodium dithionite or sodium borohydride as generally described hereinabove to afford the desired compound [D-DHC].

The representative starting drugs listed below may be derivatized in this manner to the corresponding [D-QC]⁺ and [D-DHC] compounds.

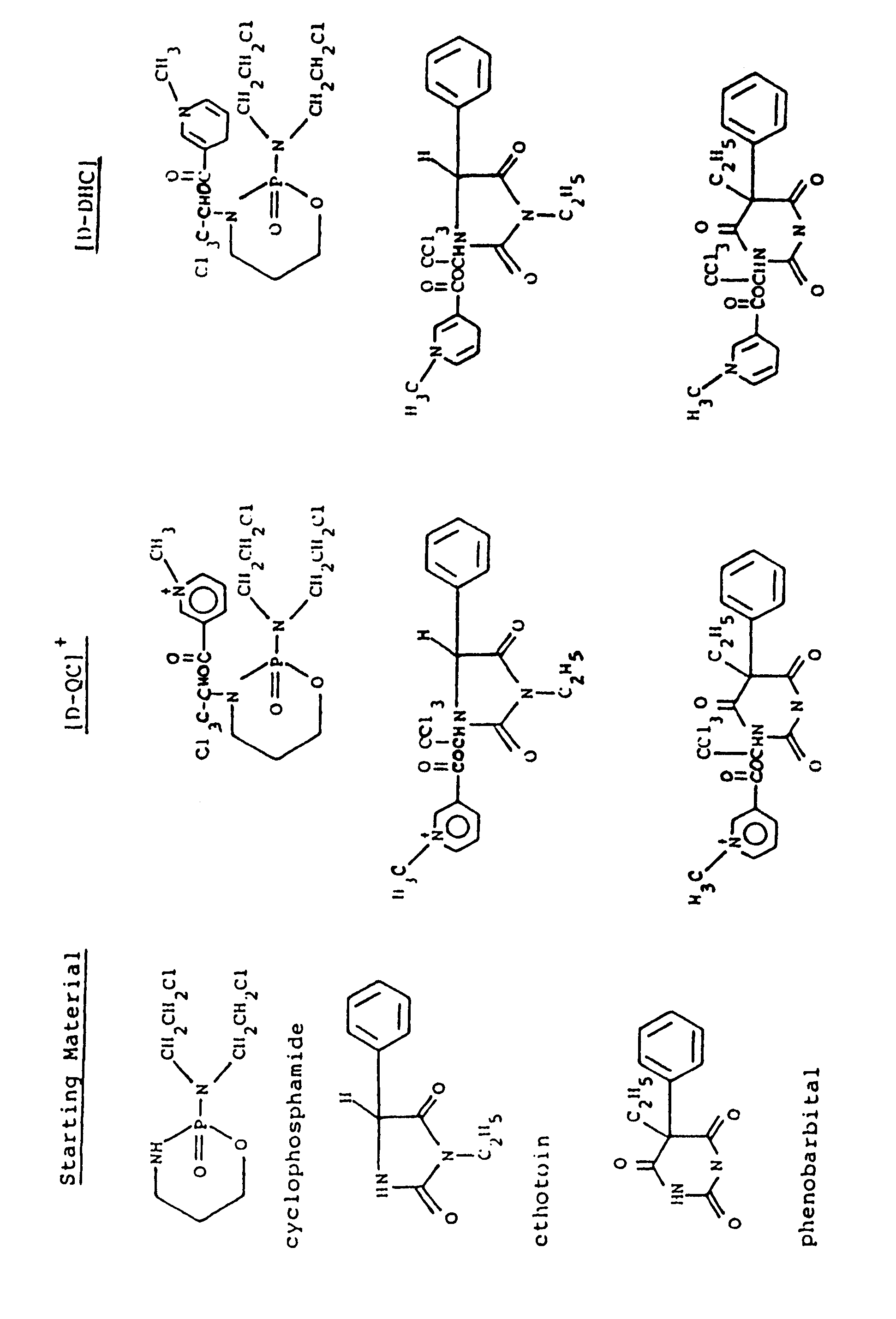

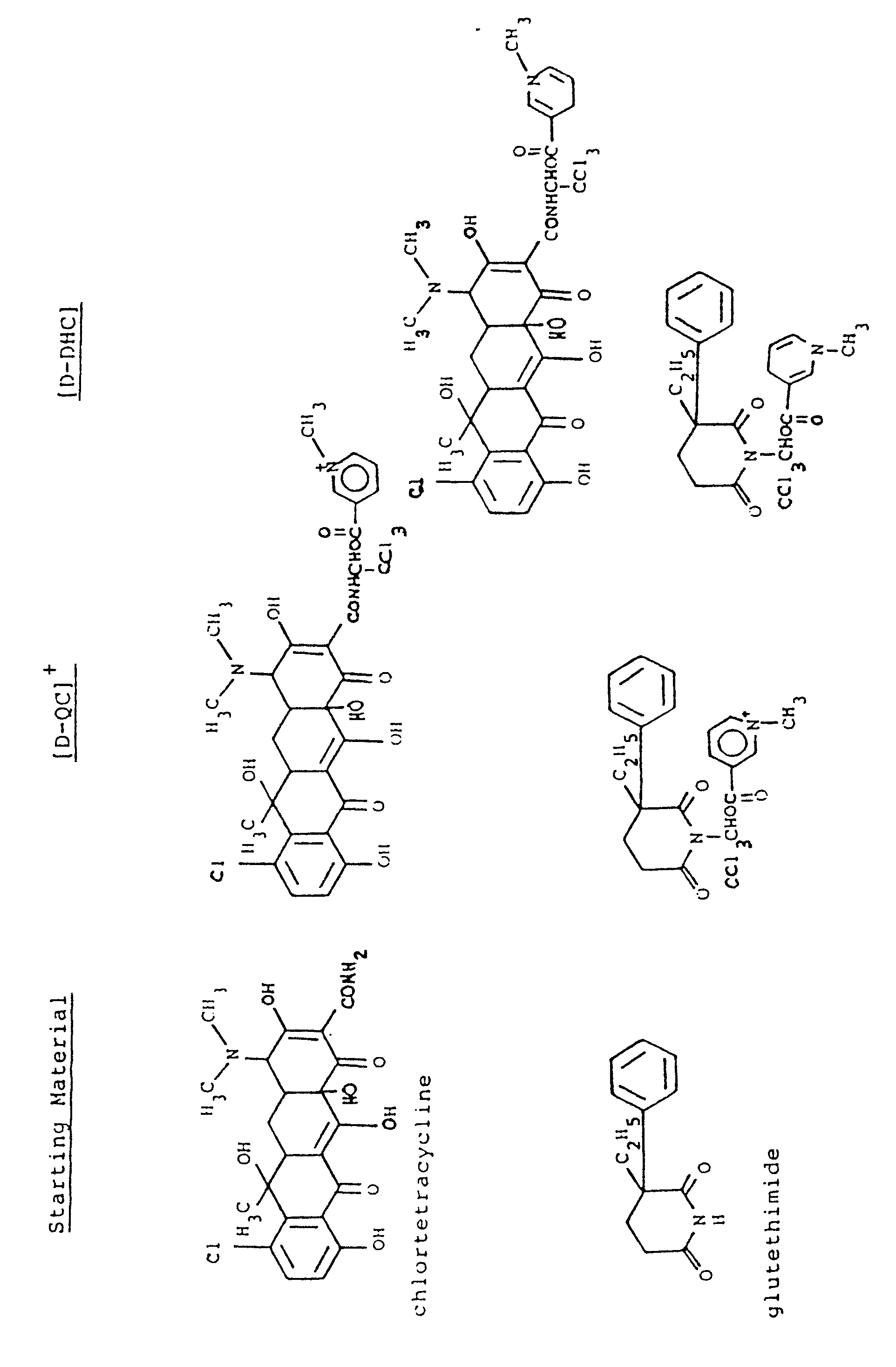

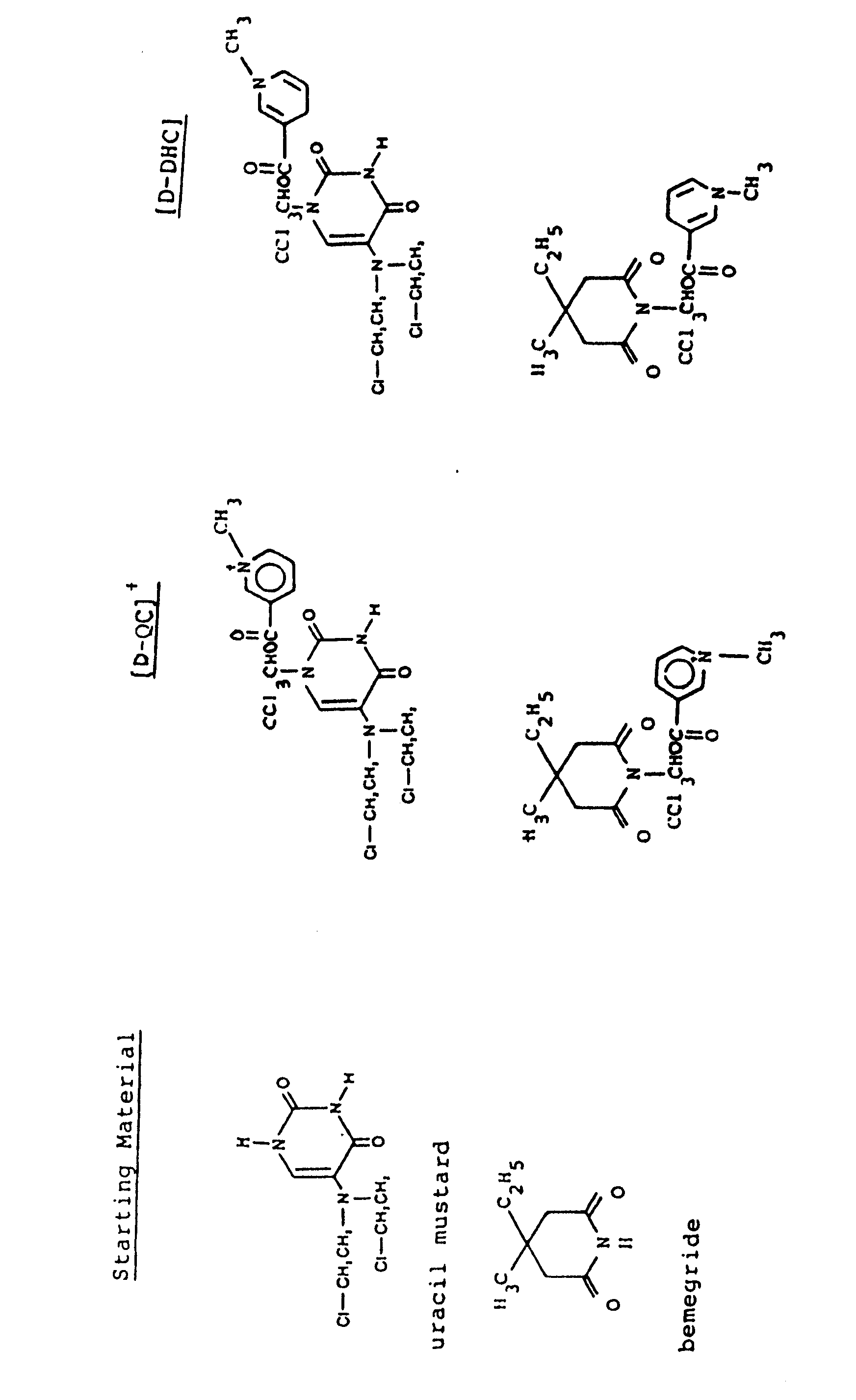

### Method B

Method A is followed, except that in the second step, a starting material of the formula 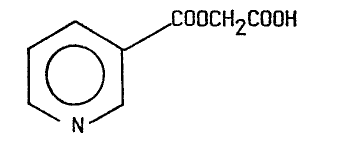 is used in place of nicotinic acid, to afford the corresponding derivatives, e.g. of the drugs listed with that method.

### II. Methods for Derivatizing -COOH Functions in Drugs

### Method C

The drug is reacted with excess alcohol of the formula 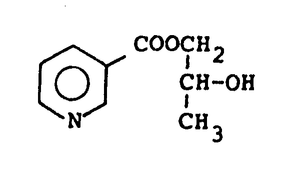 to convert the -COOH function to the corresponding 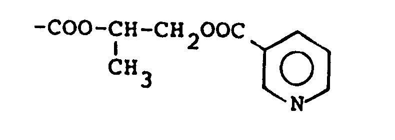 ester grouping. (That starting material may be prepared by reacting nicotinic acid with 1,2-propylene glycol in the presence of dicyclohexylcarbodiimide.) That ester is then quaternized and subsequently reduced as described above in Method A. When the drug contains more than one reactive carboxyl function, reaction conditions may be varied so that more than one carboxyl function will be converted to ester groupings.

The following representative drugs may be derivatized in this manner to the corresponding [D-QC]⁺ and [DH_{C]} compounds:

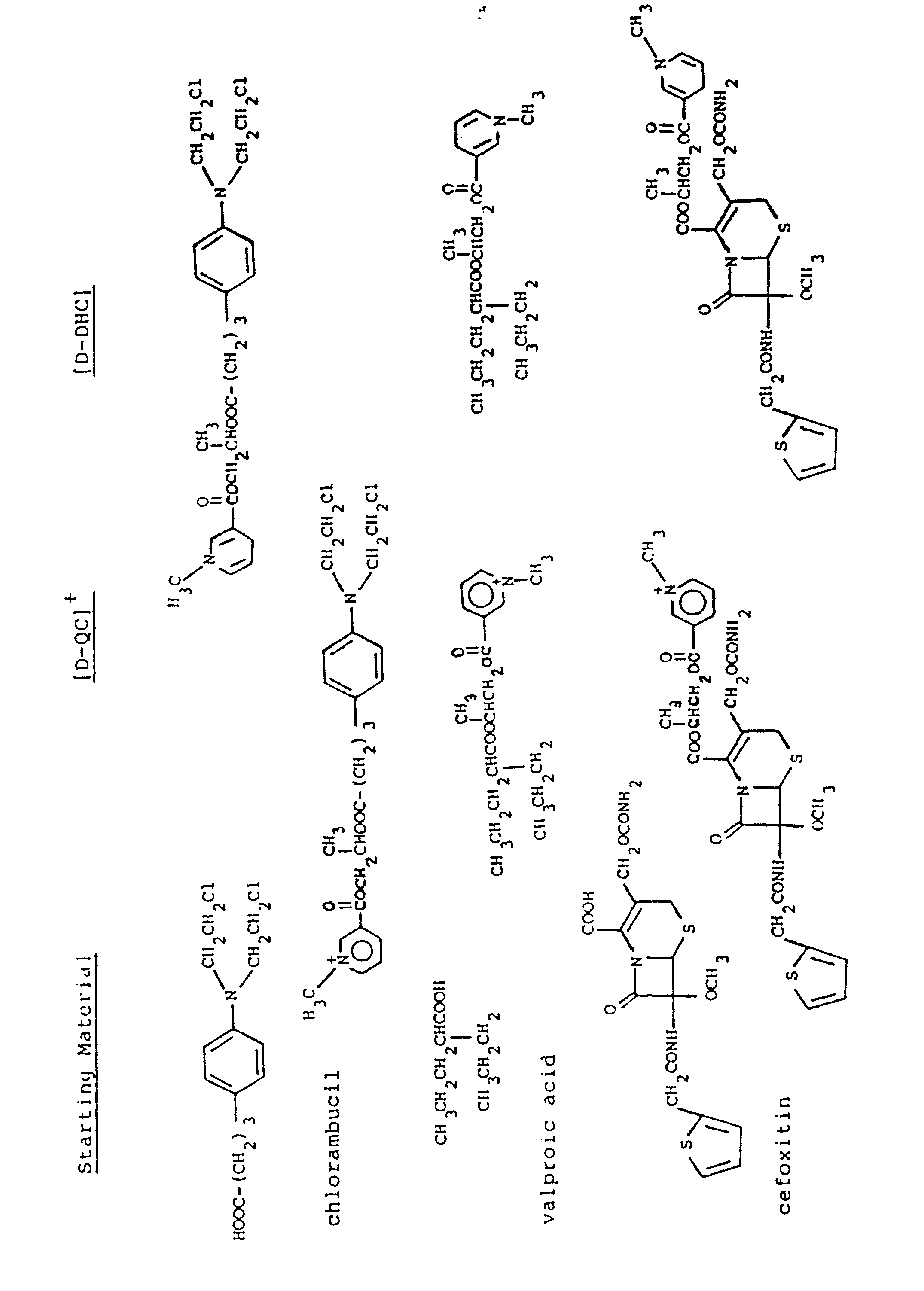

The following drugs may be similarly derivatized: 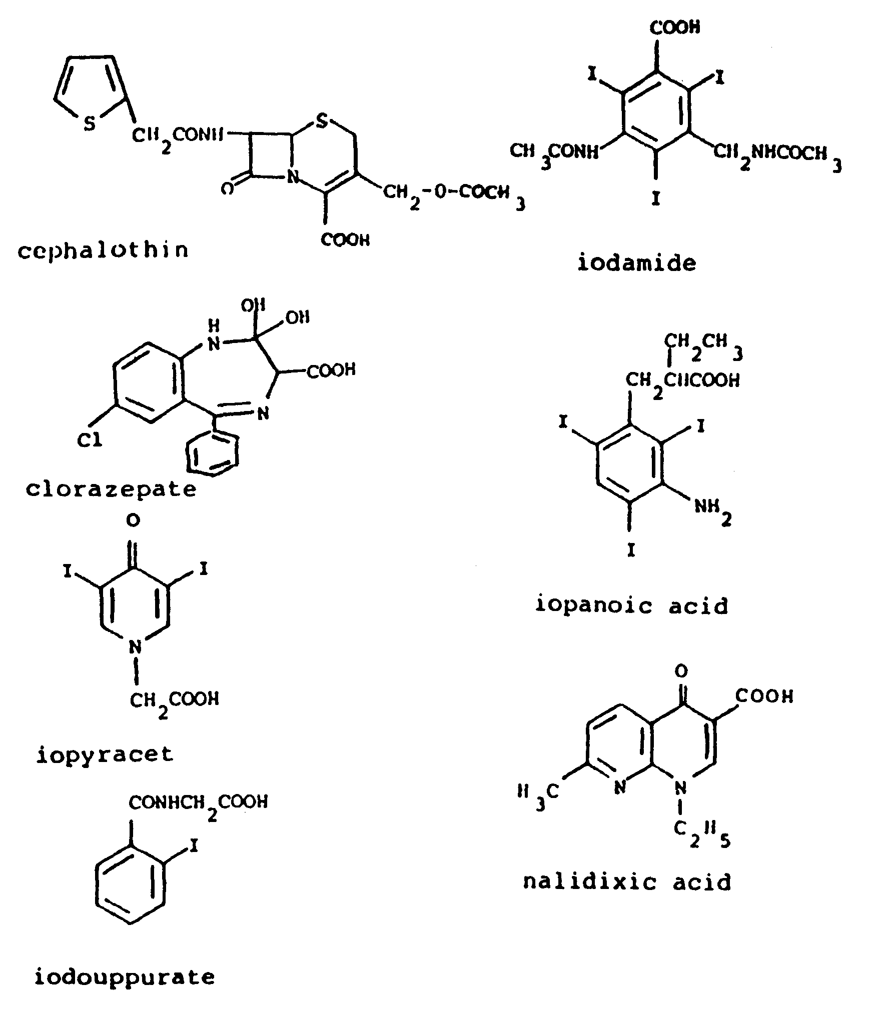 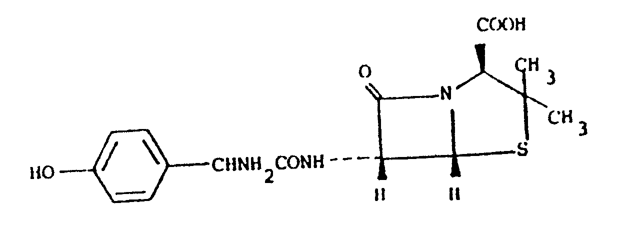 amoxicillin 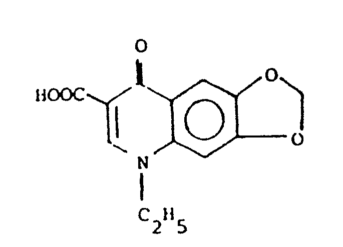 oxolinic acid 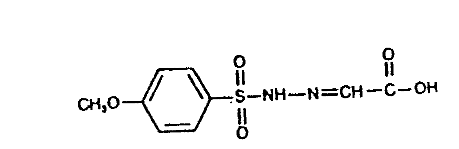 glyoxylic acid sulfonylhydrazone 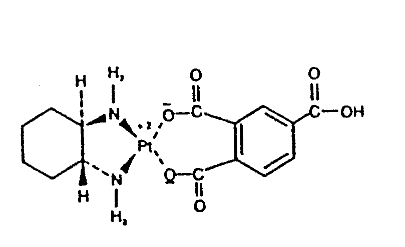 DACH 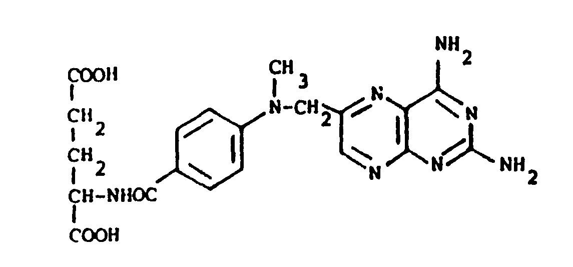 methotrexate 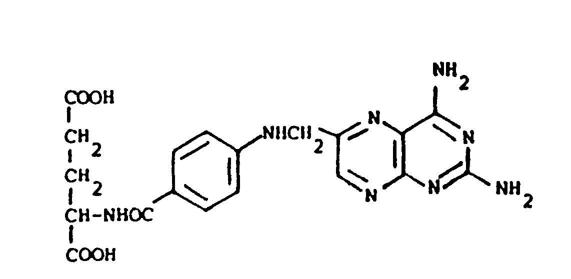 aminopterin 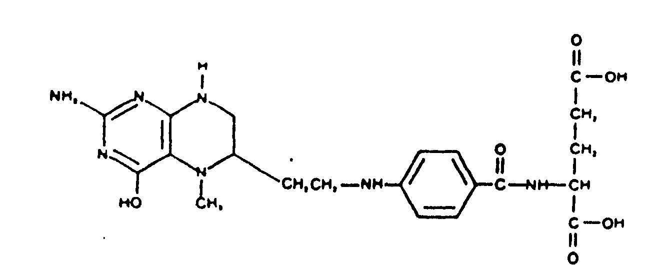 5-methyl tetrahydrohomofolic acid

### III. Methods for Salt Formation

### Method D

An ether solution of [D-DHC] is treated with an equivalent amount of anhydrous p-toluenesulfonic acid dissolved in dry ether. Mixing at room temperature is continued until the imminium salt precipitates out of solution. The salt is then removed by filtration.

Accordingly, provided hereby are not only a method and novel class of pro-prodrugs for the specific and/or target enhanced delivery to the brain of a wide variety of drug species via the bidirectional transport of the drug species into and out of the brain employing dihydropyridine 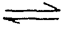 pyridinium salt carrier redox systems, but also a system providing insight into the basic transport processes (both active and passive) of, and enzymatic activities in, the blood-brain barrier, as well as into the various processes specific to the function of the brain. Again, another very significant aspect of the bioreversible redox delivery system according to this invention is the toxicity implication, for significantly reduced is systemic toxicity by accelerating the elimination of the drug/quaternary carrier system. And even central toxicity is reduced by providing for low level, sustained release of the active drug species in the brain. Low toxicity is provided both as regards the quaternary carrier and in combination with the drug.

## Claims

1. A compound adapted for the site-specific/sustained delivery of a centrally acting drug species to the brain, said compound being a compound of the formula 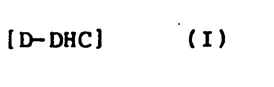 or a non-toxic pharmaceutically acceptable salt thereof, wherein [D] is a centrally acting drug species containing at least one reactive -COOH, amide or imide functional group, and [DHCI is the reduced, biooxidizable, blood-brain barrier penetrating lipoidal form of a dihydropyridine 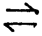 pyridinium salt redox carrier comprising a dihydropyridine ring system of the formula 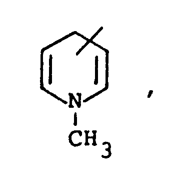 a ring carbon atom of the dihydropyridine ring system being connected via a bridging group to a reactive -COOH, amide or imide functional group in the centrally acting drug species.

2. A compound according to Claim 1 wherein the centrally acting drug species [D] contains at least one reactive amide or imide group and the redox carrier (DHC] together with the bridging group has the structural formula 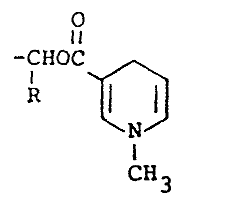 wherein R is -CC1₃,

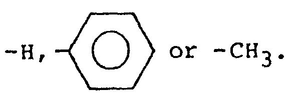

3. A compound according to Claim 1 wherein the centrally acting drug species [D] contains at least one reactive amide or imide group and the redox carrier [DHC] together with the bridging group has the structural formula 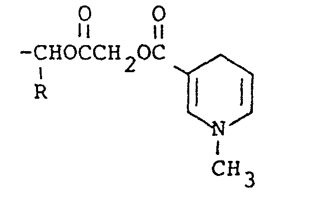 wherein R is -CCl₃, -H, 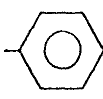 or -CH₃.

4. A compound according to Claim 1 wherein the centrally acting drug species [D] contains at least one reactive -COOH group and the bridging group connecting the redox carrier [DHC] to the drug is derived from a di- or polyhydroxy compound, at least one hydroxy function of which is attached to a reactive -COOH group in the drug and at least one other hydroxy function of which is attached via a carbonyl function to the redox carrier [DHC].

5. A compound according to Claim 4 wherein the bridging group is derived from ethylene glycol.

6. A compound according to Claim 4 wherein the bridging group is derived from propylene glycol.

7. A compound according to Claim 4 wherein the bridging group is derived from inositol.

8. A compound according to Claim 4 wherein the bridging group is derived from a sugar.

9. A compound of the formula 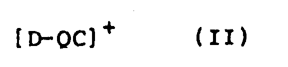 wherein [_{D]} is a centrally acting drug species containing at least one reactive -COOH, amide or imide functional group and [pC]⁺ is the hydrophilic, ionic pyridinium salt form of a dihydropyridine 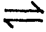 pyridinium salt redox carrier comprising a pyridinium ring system of the formula 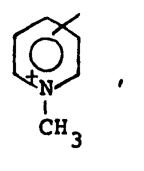 a ring carbon atom of the dihydropyridine ring system being connected via a bridging group to a reactive -_{COOH}, amide or imide functional group in the centrally acting drug species.

10. A compound according to Claim 9 wherein the centrally acting drug species [D] contains at least one reactive amide or imide group and the redox carrier [OC]⁺ together with the bridging group has the structural formula 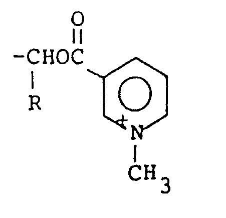 wherein R is -CC1₃, -H, 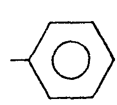 or -C^{H}₃.

11. A compound according to Claim 9 wherein the centrally acting drug species [D] contains at least one reactive amide or imide group and the redox carrier [QC]⁺ together with the bridging group has the structural formula 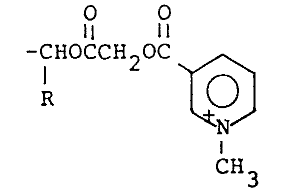 wherein R is -CC1₃. -H, 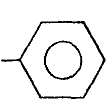 or -CH₃.

12. A compound according to Claim 1 wherein the centrally acting drug species [D] contains at least one reactive -COOH group and the bridging group connecting the redox carrier [QC]⁺ to the drug is derived from a di- or polyhydroxy compound, at least one hydroxy function of which is attached to a reactive -COOH group in the drug and at least one other hydroxy function of which is attached via a carbonyl function to the redox carrier [QC]⁺.

13. A compound according to Claim 12 wherein the bridging group is derived from ethylene glycol.

14. A compound according to Claim 12 wherein the bridging group is derived from propylene glycol.

15. A compound according to Claim 12 wherein the bridging group is derived from inositol.

16. A compound according to Claim 12 wherein the bridging group is derived from a sugar.

17. A compound according to Claim 1 or 9 wherein [D] is a central neurotransmitter, an anticancer or antitumor agent, an antiviral agent or a tranquilizer.

18. A compound according to Claim 1 or 9 wherein [D) is a sedative or hypnotic, an anticonvulsant or anti- epileptic agent, a dopaminergic agent or an antibiotic or antibacterial agent.

19. A compound according to Claim 1 or 9 wherein [D] is an amino acid or a small peptide containing 2 to 20 amino acid units.

20. A compound according to Claim 1 or 9 wherein [D) is GABA, glycine, glutamic acid, aspartic acid, neurotensin, LH_{RH}, an enkephalin, an endorphin, oxytocin M, vasopressin, 3-deazaguanine, tiazofurin, sangivamycin, PCNU, spiromustine, L-alanosine, DON, L-ICRF, 5-methyl tetrahydrohomofolic acid, glyoxylic acid sulfonylhydra- zine, DACH, SR-2555, SR-2508, bactobolin, acivicin, hydroxyurea, chlorambucil, uracil mustard, melphalan, 5-FUDR, methotrexate, aminopterin, mitomycin C, ribavirin, acyclovir, 6-azauridine, oxazepam, lorazepam, chlordiazepoxide, bromazepam, clorazepate, nitrazepam, phenytoin, ethotoin, mephenytoin, methyldopa, y-vinyl GABA, y-acetylenic GABA, phenobarbital, amobarbital, butalbital, glutethimide, aminoglutethimide, methyprylon, valproic acid, 5-hydroxy-2-n-propylpentanoic acid, 4-hydroxy-2-n-propylpentanoic acid, 3-hydroxy-2-n-propylpentanoic acid, bemegride, tryptophan, levo- dopa, ampicillin, amoxicillin, oxacillin, carbenicillin, dicloxacillin, chlortetracycline, tetracycline, methacycline, nalidixic acid, cephalexin, cephalothin, cefoxitin, clindamycin, lincomycin or oxolinic acid.
